# EUROPÄISCHE PATENTSCHRIFT

(11) **EP 4 338 768 B1**
(45) Veröffentlichungstag und Bekanntmachung des Hinweises auf die Patenterteilung: **22.04.2026**
(21) Anmeldenummer: 23195612.9
(22) Anmeldetag: 06.09.2023
(51) Int. Cl.: A61M 1/28

(54) **LECKAGEANSCHLUSS FÜR EINE DIALYSEMASCHINE, EINE DAMIT AUSGERÜSTETE LECKAGEKONSTRUKTION SOWIE EINE DIALYSEMASCHINE**
LEAKAGE CONNECTION FOR A DIALYSIS MACHINE, LEAKAGE CONSTRUCTION EQUIPPED THEREWITH AND DIALYSIS MACHINE
RACCORD DE FUITE POUR UNE MACHINE DE DIALYSE, STRUCTURE DE FUITE ÉQUIPÉE DE CELUI-CI ET MACHINE DE DIALYSE

(30) Priorität: 15.09.2022 DE 102022123642
(43) Veröffentlichungstag der Anmeldung: 20.03.2024
(73) Patentinhaber: B. Braun Avitum AG, 34212 Melsungen (DE)
(72) Erfinder: JAKOBI, Marco, 37318 Uder (DE); SCHAEFER, Oliver, 36286 Neuenstein (DE); THOMAS, Thorsten, 34613 Schwalmstadt (DE)
(74) Vertreter: Winter, Brandl - Partnerschaft mbB

(56) Entgegenhaltungen:
- EP-B1- 2 219 705
- EP-B1- 2 737 918

## Beschreibung

Die vorliegende Offenbarung betrifft einen Leckageanschluss für eine oder einer Dialysemaschine, der zum Ableiten von Leckageflüssigkeit insbesondere aus einem Filterkasten für die oder der Dialysemaschine ausgebildet ist. Die vorliegende Offenbarung betrifft weiterhin eine Leckagekonstruktion für die oder der Dialysemaschine mit einem derartigen Leckageanschluss und die Dialysemaschine mit der Leckagekonstruktion.

### Hintergrund der Offenbarung

Dialysemaschinen bedienen sich in einigen allgemein bekannten Dialyseverfahren wie beispielsweise der Hämodialyse einer Dialysierflüssigkeit, welche einem Dialysefilter zugeführt wird, um u.a. nach dem Diffusionsprinzip einem den Dialysefilter (Dialysator) durchströmenden Patientenblut bestimmte Blutbestandteile zu entziehen. Zur Vermeidung von Verunreinigungen wird die frische Dialysierflüssigkeit hierbei mittels zumindest eines Dialysierflüssigkeitsfilters gefiltert, bevor diese dem Dialysefilter zugeführt wird. Dieser Dialysierflüssigkeitsfilter ist in der Regel ein Hohlfaserfilter und dient zur Erzeugung von ultrareiner Dialysierflüssigkeit für Dialysebehandlungen.

Der technische Hintergrund des Dialysierflüssigkeitsfilters besteht darin, dass selbst wenn die Dialysemaschine ordnungsgemäß gereinigt und desinfiziert wurde, das zur Erzeugung von frischer Dialysierflüssigkeit in der Dialysemasche notwendige Permeat und das Bicarbonat-Konzentrat Quellen für eine mögliche Verunreinigung der hergestellten Dialysierflüssigkeit darstellen können. Derartige Verunreinigungen werden vom Dialysierflüssigkeitsfilter herausgefiltert.

Dialysierflüssigkeitsfilter sind in der Regel auf der einem Bediener abgewandten Rückseite der Dialysemaschine angeordnet und in einem speziellen/separaten Filterkasten innerhalb des Gehäuses der Dialysemaschine untergebracht. Solche Filterkästen besitzen normaler Weise auch einen Wartungszugang, der sich zur Rückseite der Dialysemaschine öffnet und mittels einer rückseitigen Tür verschließbar ist. Der Filterkasten ist zudem mittels einer (der Tür gegenüberliegenden) Rückwand vom übrigen Innenraum des Maschinengehäuses getrennt, an welcher Halterungen zur Aufnahme der Dialysierflüssigkeitsfilter (zumeist in Kartuschen- oder Patronenform) angebracht sind. Des Weiteren führen Zu- und Ablaufleitungen in den Filterkasten, über welche die Dialysierflüssigkeitsfilter in den Fluidkreis der frischen Dialysierflüssigkeit zwischengeschaltet werden können. Die Zu-und Ablaufleitungen haben ferner Anschlüsse, um an die Dialysierflüssigkeitsfilter lösbar angeschossen zu werden.

Grundsätzlich ist bei fluidführenden Leitungen insbesondere im Bereich von Anschlüssen mit Leckagen zu rechnen, ggf. infolge von fehlerhafter Montage, schadhaften Dichtungen oder einfach infolge von Löchern/Rissen in den Leitungen selbst. Da Dialysemaschinen u.a. auch sogenannte Bilanziereinrichtungen haben, mit deren Hilfe die Maschinenelektronik die Menge an frischer und verbrauchter Dialysierflüssigkeit erfassen und daraus ggf. die Flüssigkeitsmenge berechnen kann, die einem Patienten entzogen worden ist, besteht die Notwendigkeit, Leckagen möglichst frühzeitig zu erkennen und zu verorten, um so Mess- und/oder Berechnungsfehler beispielsweise in der Bilanziereinrichtung zu vermeiden.

### Stand der Technik

Die Druckschrift EP 2 219 705 B1 offenbart eine Maschine zur extrakorporalen Blutbehandlung, in deren unterem Bereich ein Sammel- bzw. Auffangbehälter für Leckageflüssigkeit jedweder Art angeordnet ist, die dann über einen Schlauch zu einem Leckagedetektor geleitet wird. Daher muss der Schlauch an den Sammelbehälter angeschlossen werden, wofür ein dichtender Leckageanschluss bereitgestellt werden muss. Dies ist aufwändig und kostspielig. Schwierig kann bei einem derartigen Leckageanschluss die korrekte Montage einer Dichtung zwischen dem Sammelbehälter und dem Schlauch sein. Dies gilt bei der gezeigten Maschine insbesondere, da durch die gezielte Ansammlung von Leckageflüssigkeit im Sammelbehälter eine dauerhafte Beaufschlagung der Dichtung mit Leckageflüssigkeit entsteht. Im Übrigen ist bei einer derartigen Anordnung eines Sammelbehälters zwar eine Ermittlung der Leckagemenge, nicht aber des genauen Leckageortes möglich.

### Kurzbeschreibung der Offenbarung

Aufgabe der vorliegenden Offenbarung ist es, einen Leckageanschluss, der zum Ableiten von Leckage oder auch Spülflüssigkeit von einer Öffnung einer Leckageauffangvorrichtung einer Dialysemaschine ausgebildet ist, und weiterhin eine Leckagekonstruktion einer Dialysemaschine mit einem derartigen Leckageanschluss und schließlich auch eine Dialysemaschine bzgl. der Montage des Leckageanschlusses zu vereinfachen. Dabei soll die sichere Abdichtung des Leckageanschlusses gegenüber der Leckageauffangvorrichtung ermöglicht werden.

Diese Aufgabe wird durch einen Leckageanschluss mit den Merkmalen des Anspruchs 1 und durch eine Leckagekonstruktion mit den Merkmalen des Anspruchs 7 und durch eine Dialysemaschine mit den Merkmalen des Anspruchs 13 gelöst.

Weitere vorteilhafte Ausgestaltungen sind Gegenstand der Unteransprüche.

Im Folgenden wird statt dem Begriff Leckageflüssigkeit vereinfachend der Begriff Leckage verwendet. Leckage bedeutet also in der vorliegenden Schrift Leckageflüssigkeit. Des Weiteren ist die Leckageauffangvorrichtung bevorzugt Bestandteil eines, eine Anzahl von Dialysierflüssigkeitsfiltern aufnehmenden Filterkastens, wobei die Dialysierflüssigkeitsfilter vorzugsweise an einer Filterkastenrückwand oder einer Filterkasten-Zugangstür montierbar sind.

Der Grundgedanke der vorliegenden Offenbarung besteht prinzipiell darin, einen Leckageanschluss in Form eines Anschlussadapters als eigenständiges, separates Bauteil bereitzustellen, der nach dem allgemeinen Funktionsprinzip eines Adapters einen speziell zur Fluidkopplung an einem Schlauch oder eine Leitung ausgebildeten Auslassanschluss und einen an irgendeine Art Durchlassöffnung auf Seiten einer Leckagefluid-Auffangvorrichtung anschließbaren Universal-Einlassanschluss hat oder aufweist, sodass ein Schlauch oder eine Leitung bequem und sicher an die irgendwie gestaltete Durchlassöffnung der Leckagefluid-Auffangvorrichtung anschließbar ist. Hierfür hat der Anschlussadapter wenigstens
- einen Schlauchanschlussstutzen oder Tülle, deren Form auf einen Schlauch oder Leitung abgestimmt ist,
- eine Anschlussmaske in Form einer eine Anlagefläche aufweisenden Montage-/Anlageplatte, in der ein einen Einlass bildendes Durchgangsloch ausgeformt ist, an welches die Tülle fluidangeschlossen ist und
- einen Dichtungsring oder Dichtungswulst, der/die auf der Anlagefläche angebracht oder aufgetragen ist und das Durchgangsloch in einem bestimmten Radialabstand umgibt.

Die Montage-/Anlageplatte dient somit als ein geometrisch einfach gestalteter Anschlussflansch, der lediglich an eine die Durchlassöffnung der Leckagefluid-Auffangvorrichtung aufweisende Wandung angepresst werden muss, beispielsweise mittels Schrauben, Nieten oder dergleichen Verbindungsmittel ohne dass auf die Form der Durchlassöffnung Rücksicht genommen werden muss.

Der beanspruchte, als separates Montagebauteil vorgesehene Leckageanschluss ist folglich zum Ableiten von Leckage oder auch von Spülflüssigkeit von einer Öffnung einer Leckageauffangvorrichtung einer Dialysemaschine ausgebildet. Die Leckageauffangvorrichtung kann von einer Schale oder einer Wanne oder einem Trichter oder einem Behälter oder dem Boden eines separaten Abteils wie z.B. eines Filterkastens gebildet sein. Die Leckageauffangvorrichtung hat keinen Sammelbereich, in dem Leckage oder Spülflüssigkeit aufgestaut wird. Die Öffnung ist z.B. an einer Rückwand des Abteils, beispielsweise des Filterkastens angeordnet. Der montierbare Leckageanschluss hat einen rahmen- oder gerüstarten Hauptkörper mit einer einen Leckageeinlass aufweisenden oder ausbildenden Montage-/Anlageplatte und einem Leckageauslass, wobei der Leckageeinlass und der Leckageauslass über einen im oder am Hauptkörper gebildeten Kanal (Leitung) miteinander fluidverbunden sind. Damit ist der Leckageanschluss zum Weiterleiten der Leckage oder der Spülflüssigkeit ausgebildet, welche in der Leckageauffangvorrichtung aufgefangen wurde. Der Leckageanschluss hat eine/einen am Hauptkörper und insbesondere an der Montage-/Anlageplatte befestigte (fest fixierte) umlaufende, d.h. den Leckageeinlass (vollständig) umgebende Dichtung/Dichtring/Dichtwulst, die dafür vorgesehen ist, gegen eine Fläche/Wandung der Leckageauffangvorrichtung aufgelegt/aufgedrückt zu werden. Damit ist die Montage des Leckageanschlusses an der betroffenen Leckageauffangvorrichtung vereinfacht, weil die/der Dichtung/Dichtring/Dichtwulst nicht in eine Öffnung an der Leckageauffangvorrichtung eingepasst werden muss und trotzdem ein Verlieren der Dichtung vermieden wird. Weiterhin ist die Abdichtung des Leckageanschlusses gegenüber der Leckageauffangvorrichtung optimiert, weil ein Verrutschen der Dichtung während der Montage vermieden ist und damit ein korrekter Sitz der Dichtung um die Auslassöffnung der Leckageauffangvorrichtung bzw. um den Leckageeinlass des Leckageanschlusses (Leckageanschlusselement) sichergestellt ist.

Besonders bevorzugt ist es, wenn die Dichtung eine an den Hauptkörper angespritzte PU-Schaumdichtung ist. Damit ist die Montage des Leckageanschlusses an der Leckageauffangvorrichtung weiter vereinfacht, und die sichere Abdichtung des Leckageanschlusses gegenüber der Leckageauffangvorrichtung ist weiter optimiert.

Bei einer bevorzugten konkreten Ausgestaltung ist der Leckageauslass mit einer Tülle gebildet, die vorzugsweise einstückig mit dem Hauptkörper gebildet ist, und die auch als Stutzen bezeichnet werden kann. Die Tülle ist an einem Ende an den Leckageeinlass angeschlossen und kann bevorzugt in deren Verlauf/Erstreckung eine Abwinklung insbesondere eine rechtwinklige Abwinklung aufweisen, sodass deren freier Endabschnitt sich rechtwinklig zum Leckageeinlass erstreckt. Auf die Tülle bzw. deren freier Endabschnitt kann ein Schlauch aufgeschoben werden. Damit ist der Leckageauslass (separates Leckageauslasselement) quasi als Leckageadapter ausgestaltet, mittels der die Leckageauslassöffnung der Leckageauffangvorrichtung mit dem Schlauch verbindbar ist. Die Leckageauslassöffnung der Leckageauffangvorrichtung kann somit hinsichtlich ihrer Form und Dimension nahezu beliebig ausgebildet sein, solange sich diese vollumfänglich innerhalb der von der Dichtung des Leckageauslasses umgebenden Fläche befindet.

Vorzugsweise ist die Tülle mit mindestens einem Versteifungsabschnitt oder Versteifungssteg/Rippe des Hauptkörpers stabilisiert, der die Tülle umfangsseitig umgreift. Die Rippe ist dabei im Abstand vorzugsweise Parallelabstand zur Montage-/Anlageplatte ausgerichtet und über eine Grundplatte mit dieser stoffeinstückig verbunden. Darüber hinaus ist die Rippe an ihren sich gegenüberliegenden Rippenenden jeweils umgebogen und zu der Montage-/Anlageplatte zurückgeführt, wodurch die Rippe zusammen mit der Montage-/Anlageplatte eine Art Kasten-/Gerüststruktur ausbildet, die an einer Unterseite durch die Grundplatte verschlossen und einer Oberseite offen ist. Grundplatte und Montage-/Anlageplatte bilden somit eine Art Winkelstück, welches durch die wenigstens eine Rippe ausgesteift ist.

Wenn die Tülle eine - vorzugsweise umlaufende - Schlauchdichtung hat, die einstückig an der Tülle gebildet ist, kann der Schlauch gegenüber der Tülle sicher abgedichtet werden.

Bei einer besonders bevorzugten Weiterbildung des Leckageanschlusses ist die umlaufende Dichtung des Leckageeinlasses teilweise oder vollständig von einer Anlagefläche umgeben, welche durch die Montage-/Anlageplatte gebildet wird. Damit wird eine korrekte Lage des Hauptabschnitts relativ zur betroffenen Leckageauffangvorrichtung sichergestellt, wenn diese eine entsprechende äußere Anlagefläche hat. Dies ist z.B. der Fall, wenn die Leckageauffangvorrichtung von dem Filterkasten gebildet ist und die Rückwand hat, in der die Öffnung angeordnet ist. Im Ergebnis wird die Dichtung gleichmäßig an die Öffnung der Leckageauffangvorrichtung gespannt. Damit ist die Abdichtung des Leckageanschlusses gegenüber der Leckageauffangvorrichtung weiter optimiert.

Die Anlagefläche kann an der Anlageplatte gebildet sein, wie dies vorstehend ausgeführt ist. Vorzugsweise ist die Anlageplatte mit mindestens einem Versteifungsabschnitt oder Versteifungssteg des Hauptkörpers stabilisiert. Die Tülle kann dabei derart ausgebildet sein, dass sich deren Abwinkelung bezüglich der Montage-/Anlageplatte hinter der letzten Rippe anordnet, sodass der freie Endabschnitt der Tülle quasi außerhalb des von der Rippe, der Grundplatte sowie der Montage-/Anlageplatte gebildeten Kastens längs der Rippe geführt ist.

Im montierten Zustand des Leckageanschlusses ist die Anlagefläche vorzugsweise etwa senkrecht. Dann hat der Kanal ein Gefälle. Dann kann die Leckage auf Grund der Schwerkraft durch den Leckageanschluss fließen, ohne dass sich darin Leckage sammelt.

Wenn der Kanal die wenigstens eine Krümmung oder Abwinkelung aufweist, kann die Tülle etwa parallel zur Anlagefläche oder etwa parallel zu einer durch die Anlagefläche definierten Ebene ausgerichtet sein. Damit kann Bauraum, genauer gesagt Tiefe der betroffenen Dialysemaschine eingespart werden. Dies gilt insbesondere, wenn der auf die Tülle aufgeschobene Schlauch und knickfrei bogenförmig nach unten gekrümmt ist.

Die Tülle kann in einer Ausführungsform auch nach ,unten' abgewinkelt sein, um ein störungsfreies Abfließen der Flüssigkeit in einen an die Tülle angeschlossenen Schlauch zu ermöglichen.

Die beanspruchte Leckagekonstruktion gemäß der vorliegenden Offenbarung hat die genannte Leckageauffangvorrichtung. Die Leckageauffangvorrichtung hat die genannte Öffnung, an die der Leckageeinlass des vorbeschriebenen Leckageanschlusses mittels einer Haltevorrichtung dichtend angesetzt, z.B. gespannt ist. Damit sind die bereits oben genannten Vorteile erzielt, nämlich die Montage des Leckageanschlusses an der Leckageauffangvorrichtung ist vereinfacht, weil ein Verlieren der Dichtung vermieden ist. Weiterhin ist die Abdichtung des Leckageanschlusses gegenüber der Leckageauffangvorrichtung optimiert, weil ein Verrutschen der Dichtung während der Montage vermieden ist und damit ein korrekter Sitz der Dichtung sichergestellt ist.

Wenn die Leckageauffangvorrichtung von dem Filterkasten der Dialysemaschine gebildet ist, kann die Leckage dem darin aufgenommenen Dialysierflüssigkeitsfilter einschließlich den dortigen Leitungen und Dichtungen zugeordnet werden.

Bei einer bevorzugten Weiterbildung hat die Leckageauffangvorrichtung einen Steg, der abschnittsweise zwischen eine erste Steganlage und eine zweite Steganlage des Hauptkörpers des Leckageanschlusses eingesetzt ist. **In** einer Betriebsposition der betroffenen Dialysemaschine ist der Steg etwa waagerecht, und die erste Steganlage ist eine obere Steganlage, während die zweite Steganlage eine untere Steganlage ist.

Wenn die Leckageauffangvorrichtung von dem Filterkasten gebildet ist, wird es aus fertigungstechnischen Gründen bevorzugt, wenn der Steg flächig und einstückig mit einem im Wesentlichen ebenen Boden oder Bodenabschnitt des Filterkastens gebildet ist. Der Bodenabschnitt kann gegenüber einem weiteren Bodenabschnitt des Filterkastens erhöht sein, wodurch sich ein gestufter Boden ergibt.

Vorzugsweise hat der Steg eine Ausnehmung, in die der Hauptkörper abschnittsweise eingesetzt ist. Die Ausnehmung dient als Positionierhilfe und ist derart angeordnet, dass der Leckageeinlass und damit dessen Dichtung im montierten Zustand passend zur Öffnung positioniert ist.

Im Falle der als Positionierhilfe dienenden Ausnehmung können die erste und die zweite Steganlage des Hauptkörpers jeweils aus drei zueinander angestellten Abschnitten bestehen.

Im Falle der als Positionierhilfe dienenden Ausnehmung hat diese und/oder der Hauptkörper vorzugsweise zumindest eine Einführschräge. Vorzugsweise haben die Ausnehmung und der Hauptkörper jeweils ein Paar von beidseitigen spiegelsymmetrischen Einführschrägen. Diese dienen bei der Montage des Leckageanschlusses als Einführhilfen in der Ebene des Steges, also in der Betriebsposition in waagerechter Richtung.

Die Haltevorrichtung weist vorzugsweise Schrauben auf, die sich durch Durchgangslöcher des Steges erstrecken, und die in Schraublöcher des Hauptkörpers eingeschraubt sind. Die Schraublöcher können Sacklöcher sein. Alternativ kann die Haltevorrichtung auch ein Rastmechanismus sein.

Auch senkrecht zur Ebene des Steges, also in der Betriebsposition der Dialysemaschine in senkrechter Richtung können Einführschrägen vorgesehen sein. Diese sind z.B. an den Schraublöchern des Hauptabschnitts gebildet.

Die Öffnung, der Leckageeinlass, der Kanal, die Tülle und der Schlauch können derart geformt und dimensioniert sein, dass alleine auf Grund der Schwerkraft ein Fluss von bis zu 1200 ml/min möglich ist. Damit kann die Leckagekonstruktion auch zur Spülung genutzt werden.

Die beanspruchte Dialysemaschine gemäß der vorliegenden Offenbarung umfasst die vorbeschriebene Leckageauffangvorrichtung, die z.B. von dem Filterkasten der Dialysemaschine gebildet ist. Die Leckageauffangvorrichtung umfasst die genannte Öffnung, an die der Leckageeinlass des vorbeschriebenen Leckageanschlusses mittels einer Haltevorrichtung dichtend angesetzt, z.B. gespannt ist. Damit sind auch bezüglich der Dialysemaschine die bereits oben genannten Vorteile erzielt, nämlich die Montage des Leckageanschlusses an der Leckageauffangvorrichtung ist vereinfacht, weil ein Verlieren der Dichtung vermieden ist. Weiterhin ist die Abdichtung des Leckageanschlusses gegenüber der Leckageauffangvorrichtung optimiert, weil ein Verrutschen der Dichtung während der Montage vermieden ist und damit ein korrekter Sitz der Dichtung sichergestellt ist.

Beim Stand der Technik gemäß der Druckschrift EP 2 219 705 B1 wird die Leckage nicht direkt zum Leckagedetektor weitergleitet, sondern zuvor gesammelt. Genauer gesagt hat die offenbarte Maschine eine Tragstruktur mit einer Kammer, in der die Leckage gesammelt und über einen Ablaufkanal zu einem Entleerungsbereich geführt wird. Der Entleerungsbereich befindet sich außen im Sockelbereich des Gerätes wo die Leckage vom Leckagedetektor erkannt wird.

In der Betriebsposition der Dialysemaschine gemäß der vorliegenden Offenbarung ist die Öffnung vorzugsweise an der tiefsten Stelle der Leckageauffangvorrichtung angeordnet und der Kanal hat vorzugsweise ein Gefälle, so dass die Tülle unterhalb des Leckageeinlasses angeordnet ist. Unterhalb der Tülle z.B. in einem Sockelbereich der Dialysemaschine ist ein Leckagedetektor angeordnet. Der Leckagedetektor ist über einen monoton fallenden Schlauch mit der Tülle verbunden, oder der Leckagedetektor ist unterhalb eines Ausgangs des monoton fallenden Schlauches angeordnet. Der Ausgang des Schlauches und der Leckagedetektor können also direkt oder indirekt verbunden sein. Der Schlauch kann bis in einen Erfassungsbereich ragen, wo der Leckagedetektor sitzt, oder aber der Schlauch trifft an einer beliebigen Stelle auf den Sockel, von wo aus die Leckageflüssigkeit gezielt zum Leckagedetektor fließen kann. Dann kann die Leckage auf Grund der Schwerkraft durch den Leckageadapter und den Schlauch fließen, ohne dass sich darin Leckage sammeln kann. Weiterhin ist auch im Erfassungsbereich des Leckagedetektors keine Sammelstelle für Leckage vorgesehen. Damit sind eine unmittelbare und unverzügliche Erkennung der Leckage und eine entsprechende Meldung oder eine andere Konsequenz, insbesondere ein Stopp des Flusses durch den im Filterkasten angeordneten Dialysierflüssigkeitsfilter, möglich.

Bei einer besonders bevorzugten Weiterbindung ist der Leckagedetektor derart ausgelegt, dass dieser bei einem Volumen von 100 ml nach 5 - 7 Sekunden anspricht und die Dialysemaschine den Fluss durch den im Filterkasten angeordneten Dialysierflüssigkeitsfilter stoppt.

### Kurzbeschreibung der Figuren

Figur 1 zeigt einen Teil einer Dialysemaschine in einer perspektivischen Rückansicht mit einem Filterkasten,
Figur 2 zeigt einen Teil des Filterkastens aus Figur 1 in einer perspektivischen Ansicht von innen,
Figur 3 zeigt eine Leckagekonstruktion gemäß einem Ausführungsbeispiel,
Figur 4 zeigt den Leckageadapter aus Figur 3 in einer perspektivischen Ansicht,
Figur 5 zeigt den Leckageadapter aus Figur 3 in einer weiteren perspektivischen Ansicht,
Figur 6 zeigt den Leckageadapter aus Figur 3 in einer Ansicht,
Figur 7 zeigt einen Ausschnitt des Leckageadapters aus Figur 3 in einer weiteren Ansicht,
Figur 8 zeigt einen Teil der Rückseite des Filterkastens mit dem Leckageadapter aus Figur 2 in einer Ansicht von unten, und
Figur 9 zeigt einen Teil der Rückwand mit dem Leckageadapter aus Figur 3 in einer geschnittenen Darstellung.

### Beschreibung eines bevorzugten Ausführungsbeispiels

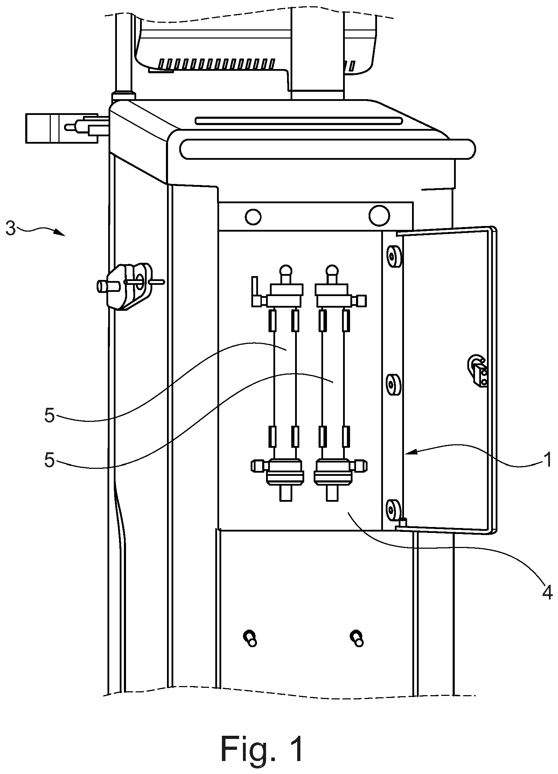

Figur 1 zeigt einen Teil einer Dialysemaschine 3 in einer perspektivischen Ansicht von hinten. An der Rückseite ist ein Filterkasten 1 gezeigt, dessen rückseitige Tür geöffnet ist. Damit ist der Blick frei auf zwei Dialysierflüssigkeitsfilter 5, die zur Erzeugung von ultrareiner Dialysierflüssigkeit für Dialysebehandlungen dient.

Weiterhin ist eine Trennwand zu erkennen, die mit Bezug zur gesamten Dialysemaschine 3 in einem mittlerne Bereich angeordnet ist. Mit Bezug zum Filterkasten 1 ist die Trennwand rückseitig und wird daher als Rückwand 4 bezeichnet.

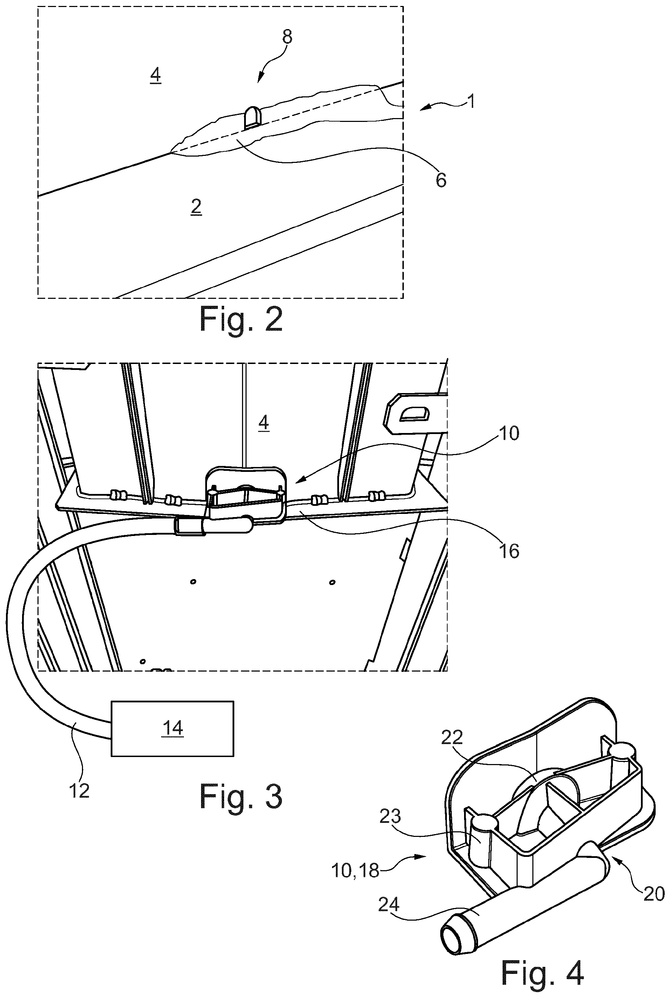

Figur 2 zeigt einen Teil des Filterkastens 1 der Dialysemaschine 3 aus Figur 1 in einer perspektivischen Ansicht von innen. Es sind ein plattenförmiger Bodenabschnitt oder Boden 2 und die plattenförmige Rückwand 4 vorgesehen. Ein zwischen dem Boden 2 und der Rückwand 4 gebildeten Eckbereich wird zu Demonstrationszwecken mit Leckage 6 in größeren unrealistischen Mengen beaufschlagt. Diese fließt unverzüglich durch eine Öffnung 8 zu einem rückseitigen Bereich des Filterkastens 1.

Figur 3 zeigt die Leckagekonstruktion gemäß einem Ausführungsbeispiel. Es sind ein Teil der Rückwand 4 des Filterkastens 1 der Dialysemaschine 3 aus Figur 2 mit einem als separater Leckageadapter 10 ausgestalteten Leckageanschluss dargestellt. Der Leckageadapter 10 ist an die in Figur 2 gezeigte Öffnung 8 angesetzt und leitet die Leckage 6 weiter zu einem Schlauch 12 der z.B. aus Silikon bestehen kann. Der monoton fallende Schlauch 12 leitet die Leckage 6 zu einem Leckagedetektor 14, der unterhalb der Öffnung 8 z.B. in einem Sockelbereich der (in Figur 1 gezeigten) Dialysemaschine 3 angeordnet sein kann. Die Leckage 6 kann auf Grund der Schwerkraft durch den Leckageadapter 10 und den Schlauch 12 fließen, ohne dass sich darin Leckage 6 sammeln kann. Weiterhin ist auch im Erfassungsbereich des Leckagedetektors 14 keine Sammelstelle für Leckage 6 vorgesehen. Damit ist eine unmittelbare und unverzügliche Erkennung der Leckage möglich, so dass der Nutzer reagieren kann und die Leckage beispielsweise durch einen Stopp des Flusses durch die im Filterkasten 1 angeordneten Dialysierflüssigkeitsfilter 5 beseitigen kann.

Der Leckageadapter 10 ist an einem Dialysemaschinen-seitigen Steg 16 befestigt, der sich in Betriebsposition der Dialysemaschine 3 etwa waagerecht oder horizontal von Rückwand 4 (mit Bezug zum Filterkasten 1) nach hinten erstreckt. Der Steg 16 ist einstückig mit dem in Figur 2 gezeigten Boden 2 gebildet. Die Rückwand 4 und der Boden 2 zusammen mit dem Steg 16 sind aus Metall gefertigt.

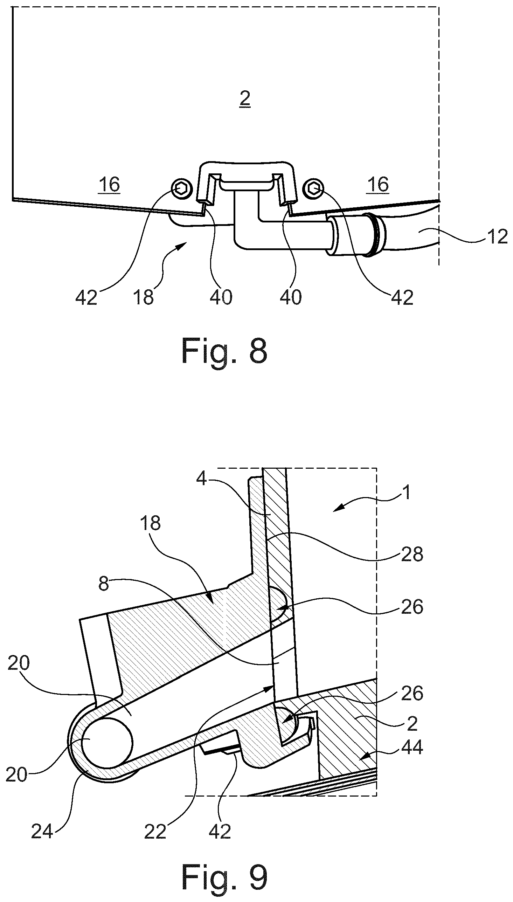

Auf diese Befestigung des Leckageadapters 10 am Steg 16 wird mit Bezug zu Figuren 8 und 9 genauer eingegangen.

Figur 4 zeigt den Leckageadapter 10 aus Figur 3 in einer perspektivischen Ansicht. Er hat eine aus Kunststoff bevorzugt im Spritzgießverfahren hergestellten Hauptkörper 18, in dem ein abgewinkelter Kanal 20 für die Leckage 6 gebildet ist. Der Kanal 20 verbindet einen Leckageeinlass 22 mit einem als Tülle 24 oder Stutzen gebildeten Leckageauslass. Auch der Kanal 20 ist monoton fallend, so dass sich die Leckage 6 auch darin nicht sammeln kann.

Weiterhin sind zwei kreiszylindrischen Abschnitte 23 des Hauptkörpers 18 zu erkennen, in denen (in Figur 4 nicht zu erkennende) als Sacklöcher ausgebildete Schraublöcher gebildet sind. Die kreiszylindrischen Abschnitte 23 sind über einen jeweiligen Versteifungsabschnitt mit dem Leckageeinlass 22 verbunden.

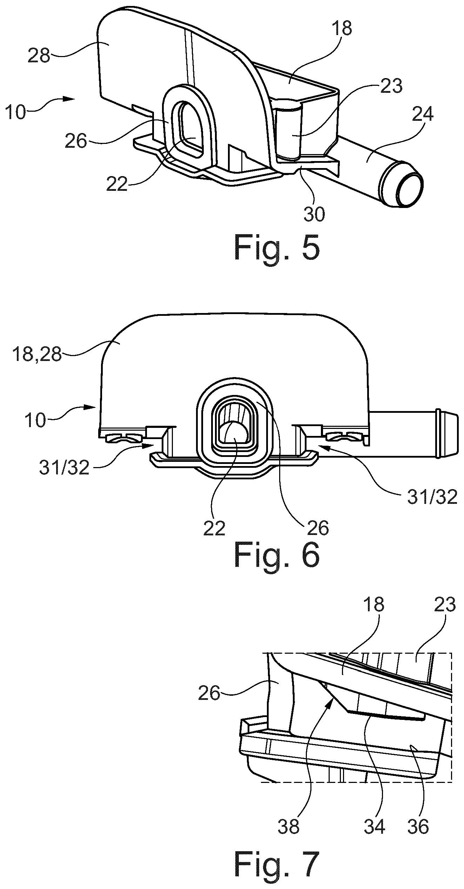

Figur 5 zeigt den Leckageadapter 10 aus Figur 3 in einer weiteren perspektivischen Ansicht. Dabei ist insbesondere eine Dichtung 26 zu erkennen, die den Leckageeinlass 22 umgibt. Die Dichtung 26 ist eine an den Hauptkörper 18 angespritzte PU-Schaumdichtung. Die PU-Schaumdichtung ist weitgehend von einer Anlagefläche 28 umgeben, die an einer Anlageplatte gebildet ist, die einstückig mit dem Hauptkörper 18 gebildet ist.

In dem (in Figur 3 gezeigten) montierten Zustand des Leckageadapters 10 umgibt die Dichtung 26 des Leckageadapters 10 die Öffnung 8 der Rückwand 4 dichtend. Eine gleichmäßige Anlage der Dichtung 26 an der Rückseite der Rückwand 4 wird durch die Anlagefläche 28 des Hauptkörpers 18 gewährleistet, die vergleichsweise großflächig an der Rückseite der Rückwand 4 anliegt.

Die Tülle 24 ist über einen Versteifungsabschnitt 30 mit dem benachbarten kreiszylindrischen Abschnitt 23 verbunden.

Figur 6 zeigt den Leckageadapter 10 aus Figur 3 in einer weiteren Ansicht. Es ist zu erkennen, dass unterhalb der Anlagefläche 28 Freiräume 31 oder Schlitze vorgesehen sind. In diese wird der in Figur 3 gezeigte Steg 16 bei der Montage des Leckageadapters 10 aufgenommen. Weiterhin sind Einführschrägen 32 am Hauptkörper 18 beidseitig der Dichtung 26 vorgesehen, die bei der Montage in waagerechter bzw. horizontaler Richtung wirken, und die dazu führen, dass die Dichtung 26 exakt vor der Öffnung 8 an der Rückseite der Rückwand 4 positioniert wird.

Figur 7 zeigt einen Ausschnitt des Leckageadapters 10 aus Figur 3 in einer weiteren Ansicht. Es sind eine erste (obere) Steganlage 34 und eine zweite (untere) Steganlage 36 zu erkennen, zwischen die bei der Montage des Leckageadapters 10 der in Figur 3 gezeigte Steg 16 abschnittsweise aufgenommen wird.

An den beiden Mündungen der in den kreiszylindrischen Abschnitten 23 gebildeten Schraublöcher ist jeweils eine Einführschräge 38 gebildet, von denen in Figur 7 nur eine Einführschräge 38 dargestellt ist. Die Einführschrägen wirken 38 in senkrechter bzw. vertikaler Richtung.

Figur 8 zeigt den Boden 2 mit dem fertig montierten Leckageadapter 10 aus den Figuren 3 bis 7 in einer Ansicht von unten. In dieser Ansicht ist im oberen Bildteil der Boden 2 des Filterkastens 1 und im unten Bildteil der einstückig damit gebildete Steg 16 dargestellt. Es ist zu erkennen, dass der Steg 16 eine Ausnehmung mit Einführschrägen 40 hat, in die der Hauptkörper 18 des Leckageadapters 10 abschnittsweise eingeschoben wird. Danach wird beim gezeigten Ausführungsbeispiel der Hauptkörper 18 und damit der Leckageadapter 10 mittels zweier Schrauben 42 gesichert.

Figur 9 zeigt einen Teil der Rückwand 4 mit dem montierten Leckageadapter 10 gemäß Figur 8 in einer geschnittenen Darstellung. In CAD-typischer Weise ist die um die Öffnung 8 umlaufende Dichtung 26 so gezeigt, als wenn sie in die ebene Rückseite der Rückwand 4 eingedrungen wäre. Es ist die Abwinkelung des Kanals 20 zu erkennen. Damit ist der Bauraumbedarf der Leckageanordnung minimiert, da der (in Figur 9 nicht gezeigte) Schlauch 12 knickfrei in einem nach unten gerichteten Bogen angesetzt wird.

Unter dem Boden 2 des Filterkastens 1 ist noch ein Teil eines Netzteilträgers 44 der Dialysemaschine 3 dargestellt.

Die Leckagekonstruktion mit der Öffnung 8 des Filterkastens 1 und mit dem Leckageeinlass 22 und mit dem Kanal 20 und mit der Tülle 24 und mit dem Schlauch 12 sind derart geformt und dimensioniert, dass alleine auf Grund der Schwerkraft der Leckage 6 oder des Spülmittels ein Fluss von bis zu 1200 ml/min möglich ist. Damit ist die Leckagekonstruktion auch zur Spülung des Filterkastens geeignet.

Der Boden 4 und die Öffnung 8 und der Leckageeinlass 22 und der Kanal 20 und die Tülle 24 und der Schlauch 12 und der Leckagedetektor 14 sind derart übereinander angeordnet, dass alleine auf Grund der Schwerkraft der Leckage 6 oder des Spülmittels deren/dessen Abführen erreicht wird, ohne dass sich irgendwo auf diesem Pfad nennenswerte Flüssigkeitsreste ansammeln können.

### Bezugszeichenliste:

- 1: Filterkasten
- 2: Boden
- 3: Dialysemaschine
- 4: Rückwand
- 5: Dialysierflüssigkeitsfilter
- 6: Leckage
- 8: Öffnung
- 10: Leckageadapter
- 12: Schlauch
- 14: Leckagedetektor
- 16: Steg
- 18: Hauptkörper
- 20: Kanal
- 22: Leckageeinlass
- 23: kreiszylindrischer Abschnitt / Aussteifungssäule
- 24: Leckageauslass / Tülle
- 26: Dichtung
- 28: Anlagefläche einer Montage-/Anlageplatte
- 30: Versteifungsabschnitt / Grundplatte
- 31: Freiraum
- 32: Einführschräge
- 34: erste Steganlage
- 36: zweite Steganlage
- 38: Einführschräge
- 40: Einführschräge
- 42: Schraube
- 44: Netzteilträger

## Patentansprüche

1. Leckageanschluss in Form eines Leckageanschluss-Adapters, der zum Ansetzen an eine Öffnung (8) einer Leckageauffangvorrichtung einer Dialysemaschine ausgebildet und ausgelegt ist, wobei der Leckageanschluss einen Hauptkörper (18) mit einem Leckageeinlass (22) und mit einem Leckageauslass (24) aufweist, die über einen im oder am Hauptkörper (18) gebildeten Kanal (20) miteinander verbunden sind, wobei der Leckageeinlass (22) in einer eine Anlagefläche (28) ausbildenden Montage-/Anlageplatte ausgebildet ist und eine Dichtung (26) aufweist, die an dem Hauptkörper (18) sowie auf der Anlagefläche (28) befestigt ist und den Leckageeinlass (22) umgibt.

2. Leckageanschluss nach Anspruch 1, wobei die Dichtung (26) eine PU-Schaumdichtung ist, die an den Hauptkörper (18) angespritzt ist.

3. Leckageanschluss nach einem der vorhergehenden Ansprüche, der ein Leckageadapter (10) ist, wobei der Leckageauslass (24) von oder an einer Tülle (24) gebildet ist, die einstückig mit dem Hauptkörper (18) gebildet ist.

4. Leckageanschluss nach einem der vorhergehenden Ansprüche, wobei die Dichtung (26) des Leckageeinlasses (22) teilweise oder vollständig von einer Anlagefläche (28) umgeben ist.

5. Leckageanschluss nach Anspruch 4, wobei der Kanal (20) gegenüber der Anlagefläche (28) geneigt oder schräg gestellt ist.

6. Leckageanschluss nach einem der vorhergehendend Ansprüche, wobei der Kanal (20) eine Krümmung oder Abwinkelung aufweist.

7. Leckagekonstruktion für eine oder einer Dialysemaschine, wobei die Leckagekonstruktion eine Leckageauffangvorrichtung aufweist, an deren Öffnung (8) mittels einer Haltevorrichtung ein Leckageanschluss nach einem der vorhergehenden Ansprüche dichtend angesetzt ist.

8. Leckagekonstruktion nach Anspruch 7 wobei die Leckageauffangvorrichtung einen Steg (16) hat, dessen Rand zwischen eine erste Steganlage (34) und eine zweite Steganlage (36) des Hauptkörpers (18) eingesetzt ist.

9. Leckagekonstruktion nach Anspruch 8, wobei der Steg (16) eine Ausnehmung hat, in die der Hauptkörper (18) abschnittsweise eingesetzt ist.

10. Leckagekonstruktion nach Anspruch 9, wobei die Ausnehmung und/oder der Hauptkörper (18) zumindest eine Einführschräge (32, 38, 40) haben/hat.

11. Leckagekonstruktion nach einem der Ansprüche 8 bis 10, wobei die Haltevorrichtung Schrauben (42) aufweist, die sich durch Durchgangslöcher des Steges (16) erstrecken, und die in den Hauptkörper (18) eingeschraubt sind.

12. Leckagekonstruktion nach einem der Ansprüche 8 bis 11, wobei die Leckageauffangvorrichtung von einem Filterkasten (1) der Dialysemaschine gebildet ist, und wobei der Steg (16) flächig und einstückig mit einem Boden (2) oder Bodenabschnitt des Filterkastens (1) gebildet ist.

13. Dialysemaschine mit einer Leckagekonstruktion nach einem der Ansprüche 7 bis 12, an die ein Leckagedetektor (14) angeschlossen ist.

14. Dialysemaschine nach Anspruch 13, wobei die Öffnung (8) an einer tiefsten Stelle der Leckageauffangvorrichtung angeordnet ist, und wobei der Leckageauslass (24) des Leckageadapters (10) unterhalb des Leckageeinlasses (22) des Leckageadapters (10) angeordnet ist, und wobei der Leckagedetektor (14) unterhalb des Leckageauslasses (24) angeordnet ist, und wobei der Leckagedetektor (14) über einen monoton fallenden Schlauch (12) mit dem Leckageadapter (10) verbunden ist, oder wobei der Leckagedetektor (14) unterhalb eines Ausgangs eines monoton fallenden Schlauches (12) angeordnet ist.

## Claims

1. A leakage connector in the form of a leakage connector adapter, which is formed and configured for attachment to an opening (8) of a leakage collecting device of a dialysis machine, wherein the leakage connector has a main body (18) with a leakage inlet (22) and with a leakage outlet (24), which are interconnected via a channel (20) formed in or on the main body (18), wherein the leakage inlet (22) is formed in a mounting/contact plate forming a contact surface (28) and comprises a seal (26) which is fixed to the main body (18) and on the contact surface (28) and surrounds the leakage inlet (22).

2. The leakage connector according to claim 1, wherein the seal (26) is a PU foam seal that is molded onto the main body (18).

3. The leakage connector according to any of the preceding claims, which is a leakage adapter (10), wherein the leakage outlet (24) is formed by or on a sleeve (24) which is formed integrally with the main body (18).

4. The leakage connector according to any of the preceding claims, wherein the seal (26) of the leakage inlet (22) is partially or completely surrounded by the contact surface (28).

5. The leakage connector according to claim 4, wherein the channel (20) is inclined or slanted with respect to the contact surface (28).

6. The leakage connector according to any of the preceding claims, wherein the channel (20) comprises a bending or angulation.

7. A leakage construction for or of a dialysis machine, wherein the leakage construction comprises a leakage collecting device, to the opening (8) of which a leakage connector according to any of the preceding claims is attached in a sealing manner via a retaining device.

8. The leakage construction according to claim 7 wherein the leakage collecting device has a land (16) whose edge is inserted between a first land contact (34) and a second land contact (36) of the main body (18).

9. The leakage construction according to claim 8, wherein the land (16) has a recess into which the main body (18) is inserted in portions.

10. The leakage construction according to claim 9, wherein the recess and/or the main body (18) has/have at least one insertion chamfer (32, 38, 40).

11. The leakage construction according to any of claims 8 to 10, wherein the retaining device comprises screws (42) extending through apertures of the land (16) and screwed into the main body (18).

12. The leakage construction according to any of claims 8 to 11, wherein the leakage collecting device is formed by a filter box (1) of the dialysis machine, and wherein the land (16) is flat and integrally formed with a bottom (2) or bottom portion of the filter box (1).

13. A dialysis machine comprising a leakage construction according to any of claims 7 to 12, to which a leakage detector (14) is connected.

14. The dialysis machine according to claim 13, wherein the opening (8) is arranged at a lowest point of the leakage collecting device, and wherein the leakage outlet (24) of the leakage adapter (10) is arranged below the leakage inlet (22) of the leakage adapter (10), and wherein the leakage detector (14) is arranged below the leakage outlet (24), and wherein the leakage detector (14) is connected to the leakage adapter (10) via a monotonously falling hose (12), or wherein the leakage detector (14) is arranged below an exit of a monotonously falling hose (12).

## Revendications

1. Raccord de fuite en forme d'adaptateur de raccord de fuite qui est configuré et conçu pour le placement sur une ouverture (8) d'un dispositif de collecte de fuite d'une machine de dialyse, dans lequel le raccord de fuite présente un corps principal (18) avec une entrée de fuite (22) et avec une sortie de fuite (24) qui sont reliées entre elles par le biais d'un canal (20) formé dans ou au niveau du corps principal (18), dans lequel l'entrée de fuite (22) est configurée dans une plaque de montage/d'appui formant une surface d'appui (28) et présente une garniture (26) qui est fixée au corps principal (18) ainsi que sur la surface d'appui (28) et entoure l'entrée de fuite (22).

2. Raccord de fuite selon la revendication 1, dans lequel la garniture (26) est une garniture de mousse PU qui est moulée par injection sur le corps principal (18).

3. Raccord de fuite selon l'une quelconque des revendications précédentes, qui est un adaptateur de fuite (10), dans lequel la sortie de fuite (24) est formée par ou au niveau d'une douille (24) qui est formée d'un seul tenant avec le corps principal (18).

4. Raccord de fuite selon l'une quelconque des revendications précédentes, dans lequel la garniture (26) de l'entrée de fuite (22) est entourée partiellement ou complètement par une surface d'appui (28).

5. Raccord de fuite selon la revendication 4, dans lequel le canal (20) est incliné ou placé en biais par rapport à la surface d'appui (28).

6. Raccord de fuite selon l'une quelconque des revendications précédentes, dans lequel le canal (20) présente une courbure ou un coude.

7. Structure de fuite pour une ou d'une machine de dialyse, dans laquelle la structure de fuite présente un dispositif de collecte de fuite, au niveau de l'ouverture (8) duquel est placé de manière étanche au moyen d'un dispositif de retenue un raccord de fuite selon l'une quelconque des revendications précédentes.

8. Structure de fuite selon la revendication 7, dans laquelle le dispositif de collecte de fuite présente une nervure (16), dont le bord est inséré entre une première installation nervurée (34) et une seconde installation nervurée (36) du corps principal (18).

9. Structure de fuite selon la revendication 8, dans laquelle la nervure (16) présente un évidement dans lequel le corps principal (18) est inséré par sections.

10. Structure de fuite selon la revendication 9, dans laquelle l'évidement et/ou le corps principal (18) présente/présentent au moins un biais d'introduction (32, 38, 40).

11. Structure de fuite selon l'une quelconque des revendications 8 à 10, dans laquelle le dispositif de retenue présente des vis (42) qui s'étendent à travers des trous traversants de la nervure (16) et qui sont vissées dans le corps principal (18).

12. Structure de fuite selon l'une quelconque des revendications 8 à 11, dans laquelle le dispositif de collecte de fuite est formé par un caisson-filtre (1) de la machine de dialyse, et dans laquelle la nervure (16) est formée à plat et d'un seul tenant avec un fond (2) ou une section de fond du caisson-filtre (1).

13. Machine de dialyse avec une structure de fuite selon l'une quelconque des revendications 7 à 12, à laquelle un détecteur de fuite (14) est raccordé.

14. Machine de dialyse selon la revendication 13, dans laquelle l'ouverture (8) est agencée à un endroit le plus profond du dispositif de collecte de fuite, et dans laquelle la sortie de fuite (24) de l'adaptateur de fuite (10) est agencée en dessous de l'entrée de fuite (22) de l'adaptateur de fuite (10) et dans laquelle le détecteur de fuite (14) est agencé en dessous de la sortie de fuite (24), et dans laquelle le détecteur de fuite (14) est relié à l'adaptateur de fuite (10) par le biais d'un tuyau (12) tombant de manière monotone ou dans laquelle le détecteur de fuite (14) est agencé en dessous d'une sortie d'un tuyau (12) tombant de manière monotone.
